# NOUVEAU FASCICULE DE BREVET EUROPEEN

(11) **EP 0 381 588 B2**
(45) Date de publication et mention de la décision concernant l'opposition: **03.05.2000**
(45) Mention de la délivrance du brevet: 26.05.1993
(21) Numéro de dépôt: 90400291.2
(22) Date de dépôt: 02.02.1990
(51) Int. Cl.: A61F 5/02, A61B 17/58

(54) **Stabilisateur inter-vertébral souple**
Intervertebraler, flexibler Stabilisator
Intervertebral flexible stabilizer

(30) Priorité: 03.02.1989 FR 8901445
(43) Date de publication de la demande: 08.08.1990
(73) Titulaire: Bréard, Francis Henri, F-75014 Paris (FR); Graf, Henry, F-69002 Lyon (FR)
(72) Inventeur: Bréard, Francis Henri, F-75014 Paris (FR); Graf, Henry, F-69002 Lyon (FR)
(74) Mandataire: Kédinger, Jean-Paul

(56) Documents cités:
- EP-A- 0 140 790
- EP-A- 0 145 492
- EP-A- 0 260 970
- EP-A- 0 322 334
- EP-B- 0 041 111
- WO-A-88/07357
- DE-A- 2 618 344
- DE-A- 2 821 678
- DE-A- 3 132 520
- FR-A- 1 240 313
- FR-A- 2 275 679
- GB-A- 2 084 468
- US-A- 51 954
- US-A- 4 743 260
- US-A- 4 773 910
- US-A- 4 790 850
- Senegas et al: "Le recalibrage du canal lombaire, alternative à la laminectomie dans le traitement des sténoses du canal lombaire", Revue de chirurgie Orthopédique, 1988, 75, 15-22
- "Las inestabilidades de muneca", Dr. J. E. Beltràn (traduction en français)
- E.S.S. European Spine Society, 6th Annual Meeting, Abstract Book, Noordwijk aan Zee - The Netherlands, 14-16 September 1995, page 8: Biomechanical efficacy of semirigid spine fusion instrumentation
- Mechanical and bioilogical properties of the GORETEX expanded PTFE prosthetic ligament
- Langenscheidts Handwörterbuch
- Orthopädisch-chirurgischer Operationsatlas, Band III, 1974
- "Anatomie, Atlas commenté d'anatomie humaine pour étudiants et praticiens", pages 55-59, C. Cabrol, deuxième edition 1980

## Description

La présente invention se rapporte à un stabilisateur inter-vertébral destiné à être mis en place entre au moins deux vertèbres pour corriger des défauts du rachis.

Les stabilisateurs inter-vertébraux utilisés à l'heure actuelle pour réduire les effets souvent douloureux des maladies du rachis, comme les scolioses, les hernies discales ou les instabilités lombaires, sont des plaquettes ou des tiges métalliques que l'on fixe aux vertèbres ou à leurs apophyses épineuses, le long du tronçon affecté du rachis, ce qui a pour inconvénient d'immobiliser complètement les vertèbres et donc de limiter ou même d'interdire tout mouvement de flexion ou de torsion du tronc du patient.

La présente invention se propose de remédier à cet inconvénient et, pour ce faire, elle a pour objet un stabilisateur inter-vertébral d'un type nouveau, qui se caractérise en ce qu'il comprend un ou plusieurs ligament(s) souple(s), à flexibilité omnidirectionnelle, muni(s) de moyens d'accrochage respectivement associé(s) à deux organes de retenue implantables chacun dans une vertèbre respective.

Avantageusement, le ou l'un au moins des ligaments souples présente la forme générale d'une boucle fermée ou en variante est muni d'un anneau ou d'une boucle fermée à chacune de ses extrémités, les extrémités de chaque ligament constituant alors lesdits moyens d'accrochage, par lesquels celui-ci peut être accroché à des organes de retenue, chacun de ceux-ci, de préférence constitués par une vis, présentera avantageusement une tête libre pour l'accrochage d'une extrémité respective du ligament correspondant.

On dispose ainsi d'un stabilisateur inter-vertébral souple ou semi-élastique qui, suivant son mode d'implantation, d'un côté seulement ou des deux côtés des apophyses épineuses des vertèbres touchées, sur la face avant ou la face arrière de ces dernières, avec un seul ligament ou plusieurs montés en chaîne ou mutuellement croisés, permet de compenser toutes sortes de défauts ou déformations du rachis en agissant en compression. Simultanément, et grâce à la flexibilité omnidirectionnelle du ou des ligaments utilisés, le stabilisateur selon l'invention autorise un mouvement suffisant de distraction et/ou de rotation des vertèbres pour ne pas gêner le patient dans ses mouvements de flexion ou de torsion du tronc.

A côté de cet avantage principal, le stabilisateur inter-vertébral selon l'invention est d'un montage extrêmement simple, puisqu'il suffit d'engager les extrémités de chaque ligament en forme de boucles ou d'anneaux respectivement autour, par exemple, de deux vis préalablement implantées dans les vertèbres.

Pour éviter tout glissement du ligament après sa mise en place, autour des organes de retenue, tels que des vis, il est en outre avantageusement prévu, sur chaque organe de retenue, une coiffe amovible surdimensionnée radialement par rapport à sa tête et s'adaptant sur celle-ci. On obtiendra le même effet en munissant en variante la tête de chaque organe de retenue d'une saillie latérale d'arrêt du ligament.

Plusieurs modes de réalisation du stabilisateur inter-vertébral selon l'invention, vont maintenant être décrits plus en détails, mais sans caractère limitatif, en référence aux dessins annexés sur lesquels :
- la figure 1 représente une vue latérale en coupe partielle du stabilisateur inter-vertébral selon le premier mode de réalisation de l'invention ;
- les figures 2a et 2b illustrent des variantes de réalisation du stabilisateur de la figure 2 ;

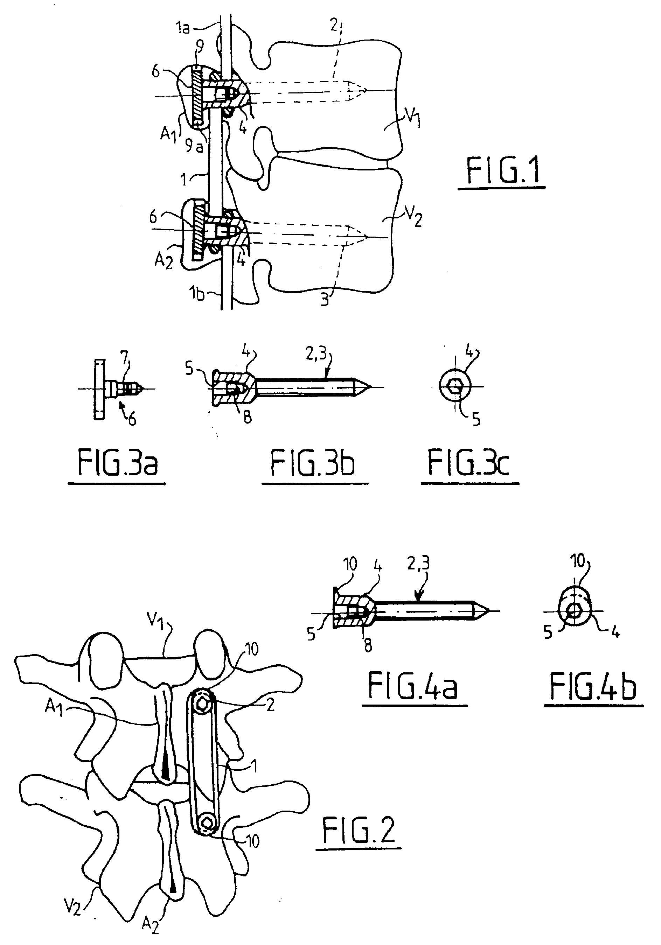

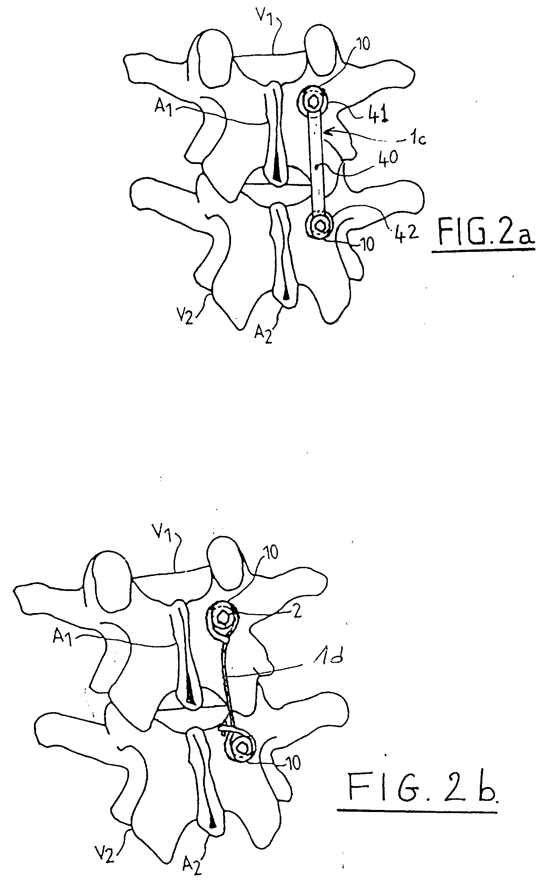

- la figure 2 est une vue de face d'un stabilisateur inter-vertébral conforme au deuxième mode de réalisation de l'invention ;
- les figures 3a, 3b et 3c illustrent l'un des organes de retenue du ligament du stabilisateur de la figure 1 ;
- les figures 4a et 4b montrent respectivement, en vue longitudinale en coupe et en vue de face, l'un des organes de retenue du stabilisateur de la figure 2 ;

La figure 1 représente deux vertèbres voisines V1, V2 du rachis d'un patient, réunies par un stabilisateur inter-vertébral selon l'invention, qui se compose d'un ligament souple 1 en forme de boucle fermée et de deux vis 2, 3 implantées chacune dans une vertèbre respective, pour retenir entre elles le ligament 1, celui-ci étant simplement engagé autour des têtes élargies cylindriques 4 des vis, émergeant des vertèbres. Le ligament 1 est un ligament artificiel en "Dacron" (marque déposée) ou tout autre matière plastique souple, à flexibilité omnidirectionnelle.

Comme le montre mieux les vues en coupe longitudinale et de face des figures 3b et 3c, chaque vis comporte, dans sa tête 4, un perçage axial borgne 5, de section hexagonale, dans lequel on peut engager une clé à six pans pour implanter la vis dans la vertèbre correspondante.

Après la mise en place du ligament 1 autour des vis ainsi implantées, on ferme le perçage 5 de chacune d'elles à l'aide d'une coiffe ou bouchon plat circulaire 6, représenté seul sur la figure 3a, ce bouchon, de diamètre nettement supérieur à celui de la tête 4 de la vis, venant se visser par un picot central 7 à bout fileté, dans un taraudage 8 débouchant dans le fond du perçage 5. Pour réaliser ce vissage, on utilisera une clé spéciale coopérant avec deux encoches 9,9a formées sur le pourtour de chaque bouchon. En variante, chaque coiffe ou bouchon 6 peut être muni d'un évidement central hexagonal et être vissé dans la tête 4 de la vis correspondante à l'aide d'une clé à six pans s'engageant dans cet évidement hexagonal. En saillant radialement tout autour des têtes respectives 4 des vis, les deux bouchons 6 empêchent tout glissement du ligament 1 hors de ces dernières, comme l'illustre la figure 1.

Les bouchons 6 présentent un intérêt particulier lorsque le stabilisateur inter-vertébral selon l'invention comprend plusieurs ligaments souples supplémentaires, tels que représentés en 1a et 1b sur la figure 1, montés en chaîne avec le premier 1, sur les vertèbres précédant et suivant les deux représentées V₁,V₂, à l'aide d'un même nombre de vis supplémentaires de retenue.

Dans le cas illustré par la figure 2, où l'on n'aura à interposer qu'un ligament 1 en forme de boucle fermée entre seulement deux vertèbres successives V₁,V₂, on peut en variante doter la tête 4 de chaque vis 2 ou 3 d'une saillie latérale 10 de hauteur convenable comme représenté de profil et de face sur les figures 4a et 4b. Dans ce cas, il faudra tout d'abord implanter les deux vis 2,3 en orientant leurs saillies 10 l'une en face de l'autre, pour pouvoir ensuite glisser sans entrave le ligament 1 autour de leur tête 4, après quoi on fera subir aux vis un demi-tour supplémentaire pour placer les saillies 10 dans leur position de retenue du ligament, représentée sur la figure 2.

Le stabilisateur inter-vertébral selon l'invention permet, selon son positionnement, de combattre de nombreuses maladies douloureuses, affectant le rachis. Celui des figures 1 et 2 est mis en place sur la face arrière des vertèbres V₁,V₂ et d'un côté seulement de leurs apophyses épineuses A₁,A₂, mais on peut aussi suivant le type de maladie à traiter, utiliser deux stabilisateurs conformes à l'invention, montés de part et d'autre des apophyses A₁,A₂ ou en croix entre les deux vertèbres V₁,V₂, sur la face avant ou la face arrière de celles-ci.

Selon une variante de réalisation du stabilisateur de la figure 2, illustrée par la figure 2a, le ligament 1c présente la forme d'un simple segment 40 dont les deux extrémités sont chacune reliées à un anneau métallique 41 ou 42, par lequel le ligament peut être accroché à la tête 4 d'une vis de retenue respective 2 ou 3.

Selon une autre variante illustrée par la figure 2b, le ligament 1d, en forme de boucle fermée, est introduit à l'intérieur de lui-même pour former une première boucle d'extrémité 43 s'engageant autour d'un organe de retenue implantée dans une vertèbre respective V₁. A hauteur de sa seconde extrémité, le ligament 1d est passé autour de l'organe de retenue implantée dans la vertèbre inférieure V₂, puis reintroduit dans lui-même pour, par l'intermédiaire d'un goujon d'arrêt 44, former une seconde boucle d'extrémité 45. Il va de soi également qu'un montage en chaîne de plusieurs ligaments, comme sur la figure 1, est possible avec les modes de réalisation des figures 2a et 2b, à l'aide de vis de retenue préalablement implantées.

Les vis 2,3 peuvent quant à elles être remplacées par tout autre organe de retenue implantable dans une vertèbre et muni d'une tête libre pour l'accrochage d'une extrémité du ligament.

Il convient encore de préciser que les vis ou autres organes de retenue 2,3, leurs bouchons 6 seront de préférence réalisées en un alliage métallique biocompatible.

## Revendications

1. Stabilisateur inter-vertébral composé d'un ensemble d'un ou plusieurs ligament(s) muni(s) de moyens d'accrochage et respectivement associé(s) à deux organes de retenue (2, 3) implantables chacun dans une vertèbre respective (V1, V2), l'ensemble étant destiné à être mis en place entre au moins deux vertèbres successives, le ou les ligament(s) étant souple(s) (1, 1a, 1b, 1c, 1d) à flexibilité omnidirectionnelle, caractérisé en ce que :
- le ou l'un au moins des ligaments souples (1, 1a, 1b) présente la forme générale d'une boucle fermée dont les deux extrémités forment lesdits moyens d'accrochage,
- le ou l'un au moins des ligaments (1c, 1d) est muni, à chacune de ses extrémités, d'un anneau (41, 42) ou d'une boucle fermée (43, 45) constituant lesdits moyens d'accrochage.

2. Stabilisateur inter-vertébral selon la revendication 1 dont chacun des ligaments, muni de moyens d'acrochage, est associé à deux organes de retenue, caractérisé en ce que chaque organe de retenue (2,3) présente une tête libre (4) pour l'accrochage d'une extrémité respective du ligament correspondant (1, 1a, 1b, 1c, 1d).

3. Stabilisateur inter-vertébral selon la revendication 2, caractérisé en ce que chaqun des organes de retenue (2,3) comprend une coiffe amovible (6), surdimensionnée radialement par rapport à sa tête (4) et s'adaptant sur celle-ci.

4. Stabilisateur inter-vertébral selon la revendication 2, caractérisé en ce que la tête de chaque organe de retenue (2, 3) est munie d'une saillie latérale (10) d'arrêt du ligament.

## Claims

1. An intervertebral stabiliser consisting of a set of one or more ligaments fitted with a means of attachment corresponding respectively to two anchor pins (2, 3) each of which may be implanted in its respective vertebra (V1, V2). The whole is intended to he placed in position between at least two consecutive vertebrae, the ligament or ligaments being souple (1, 1a, 1b, 1c, 1d) in all directions and is characterised by the fact that: :
- at least one of the souple ligaments (1, 1a, 1b) has the general shape of a closed loop whose two ends form the above-mentioned means of attachment,
- at least one of the souple ligaments (1c, 1d) is fitted at each end with a band (41, 42) or a closed loop (43, 45) forming the above-mentioned means of attachment.

2. An intervertebral stabiliser as described in Claim 1 in which each of the ligaments, fitted with a means of attachment, is used with two anchor pins characterised by the fact that each such anchor (2, 3) possesses a free head (4) to which can be attached the appropriate end of the corresponding ligament (1, 1a, 1b, 1c, 1d).

3. An intervertebral stabiliser as described in Claim 2 and characterised by the fact that each of the anchor components (2, 3) includes a removable cap (6) of greater diametric radius than the above-mentioned head (4) and intended to be fitted over the latter.

4. An intervertebral stabiliser as described in Claim 2 and characterised by the fact that the head of each anchor component (2, 3) is fitted with a lateral ridge (10) intended to keep the ligament in place.

## Patentansprüche

1. Intervertebraler Stabilisator, bestehend aus einem oder mehreren Ligamenten, die mit Befestigungen versehen und jeweils mit zwei Haltevorrichtungen (2, 3), verbunden sind, von denen jedes in einen Wirbel (V1, V2) implantiert werden kann, wobei die gesamte Vorrichtung dazu bestimmt ist, zwischen mindestens zwei aufeinanderfolgenden Wirbeln angebracht zu werden, das oder die Ligamente beweglich (1, 1a, 1b, 1c, 1d) ist/sind und in alle Richtungen gebogen werden kann/können und dadurch gekennzeichnet ist, daß :
- mindestens eines der beweglichen Ligamente (1, 1a, 1b) die Form einer geschlossenen Schleife aufweist, deren beide Enden die erwähnten Befestigungen bilden,
- mindestens eines der beweglichen Ligamente (1c, 1d) an beiden Enden einen Ring (41, 42) oder eine geschlossene Schleife (43, 45) aufweist, der/die die erwähnten Befestigungen bilden.

2. Intervertebraler Stabilisator entsprechend dem Patentanspruch 1, bei dem jedes der mit Befestigungen versehenen Ligamente mit zwei haltevorrichtungen verbunden ist, dadurch gekennzeichnet, daß jede Haltevorrichtung (2,3) ein freies Oberteil (4) zur Befestigung des jeweiligen Endes des entsprechenden Ligaments (1, 1a, 1b, 1c, 1d) aufweist.

3. Intervertebraler Stabilisator entsprechend dem Patentanspruch 2, dadurch gekennzeichnet, daß jede dieser Haltevorrichtungen (2, 3) eine abnehmbare Abdeckung (6) besitzt, die im Verhältnis zu ihrem Oberteil (4) radial größer ist und auf das Oberteil paßt.

4. Intervertebraler Stabilisator entsprechend dem Patentaspruch 2, dadurch gekennzeichnet, daß das Oberteil jeder Haltevorrichtung (2, 3) mit einer seitlichen Ausbuchtung (10) versehen ist, die ein Abgleiten des Ligaments verhindert.
